# EUROPEAN PATENT SPECIFICATION

(11) **EP 0 573 542 B1**
(45) Date of publication and mention of the grant of the patent: **15.10.1997**
(21) Application number: 92906664.5
(22) Date of filing: 27.02.1992
(51) Int. Cl.: C07D 233/64, A61K 31/415, C07D 233/94, C07D 233/95, C07D 233/90, C07D 233/68, C07D 401/04, C07D 401/12

(54) **IMIDAZOLE-DERIVATIVES HAVING ANTAGONISTIC ACTIVITY ON THE HISTAMINE H3-RECEPTOR**
IMIDAZOL-DERIVATE MIT ANTAGONISTISCHER AKTIVITÄT AUF DEN HISTAMIN-H3-RECEPTOR
DERIVES D'IMIDAZOLES POSSEDANT UNE ACTIVITE ANTAGONISTE SUR LE RECEPTEUR DE L'HISTAMINE H3

(30) Priority: 27.02.1991 NL 9100365; 22.10.1991 NL 9101764
(43) Date of publication of application: 15.12.1993
(73) Proprietor: SEED CAPITAL INVESTMENTS( SCI) B.V., 3527 GA Utrecht (NL)
(72) Inventor: TIMMERMAN, Hendrik, NL-2253 VM Voorschoten (NL); VAN DER GOOT, Henderikus, NL-2133 GW Hoofddorp (NL)
(74) Representative: Land, Addick Adrianus Gosling
(86) International application number: NL9200041
(87) International publication number: WO9215567

(56) References cited:
- EP-A- 0 041 359
- EP-A- 0 129 033
- EP-A- 0 177 808
- EP-A- 0 262 448
- WO-A-87/07891
- DE-A- 2 052 692
- DE-A- 2 433 625
- FR-A- 2 311 536
- US-A- 4 262 125
- Journal of Medicinal Chemistry, 1990, vol. 33, pages 4-11
- Comprehensive Medicinal Chemistry, vol.3, Membranes & Receptors, pages 412-413,1990, Pergamon Press

## Description

The invention relates to novel imidazole-derivatives. The invention in particular relates to novel imidazole-derivatives having antagonistic activity on the histamine H₃-receptor. More in particular it relates to isothiourea- and guanidine- derivatives. The invention further relates to the synthesis of such compounds, a pharmaceutical composition comprising such compounds or pharmacological acceptable salts thereof, the use of the compounds as agents with antagonistic activity on the histamine H₃-receptor or for preparing a pharmaceutical composition.

In addition to the already longer known histamine H₁- and H₂-receptors there is also a third type histamine-receptor present in the human body, the so-called H₃-receptor. De H₃-receptor is a presynaptic receptor, i.e. it is located on a cell releasing histamine and stimulation of the receptor leads to inhibition of the histamine-release. Furthermore stimulation of the H₃-receptor influences also the release of other neurotransmitters, such as e.g. serotonine and acetylcholine. H₃-receptors are located in the central and peripheral nervous system, the lung tissue, the intestine and probably also in the spleen, the skin and the gastro-intestinal tract. A number of compounds having an effect on H₃-receptors has already been described. For a review see Schwartz et al., Agents and Actions 30, 1/2 (1990) p. 13-23.

Chemical compounds can stimulate or inhibit the histamine H₃-receptor (Timmerman, J. Med. Chem. 33, p. 4-11 (1990)). Now it has been found that derivatives represented by the general formula: 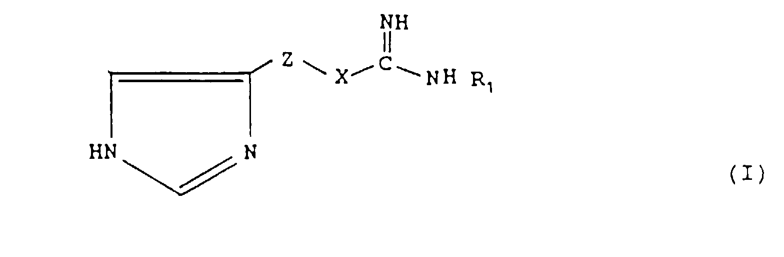 wherein the substituents are as defined in claim 1, and wherein aryl is phenyl, naphthyl, or, pyridyl, as well as the compounds S-[2-(imidazolyl-4-yl)ethyl]-N-(2-phenylethyl)isothiourea, N-cyclohexylmethyl-S-[3-(4(5)-imidazolyl) propyl]isothiourea, S-[3-(4(5)-imidazolyl) propyl]-N-(4-chlorobenzyl)isothiourea and S-[3-(4(5)-imidazolyl)propyl]-N-4-iodophenylethyl)isothiourea may be used for the preparation of pharmaceutical compositions having antagonistic activity on the histamine H₃-receptor.

Antagonistic activity is in particular shown by compounds S-[2-(imidazol-4-yl) ethyl]-N-(2-phenylethyl)isothiourea, N-benzyl-S-[3-(4(5)-imidazolyl)propyl]isothiourea, S-[3-(4(5)-imidazolyl)propyl]-N-(2-phenylethyl)isothiourea, S-[3-(4(5)-imidazolyl)propyl]-N-(3-phenylethyl)isothiourea, S-[3-(4(5)-imidazolyl)propyl]-N-(3-phenylbutyl)isothiourea, S-[3-(4(5)-imidazolyl)propyl]-N-(4-chlorobenzyl)isothiourea, N-cyclohexylmethyl-S-[3-(4(5)-imidazolyl)propyl]isothiourea and S-[3-(4(5)-imidazolyl)propyl]-N-(4-iodophenylethyl)isothiourea.

Another compound showing strong antagonistic activity is N-[2-(benzylthio)ethyl]-S-[3-(imidazol-4-yl)propyl]isothiourea.

Compounds of formula I can in general be synthesized in a for analogous compounds known manner. Favourable methods for synthesizing consist in condensation of a imidazole-compound of the general formula: 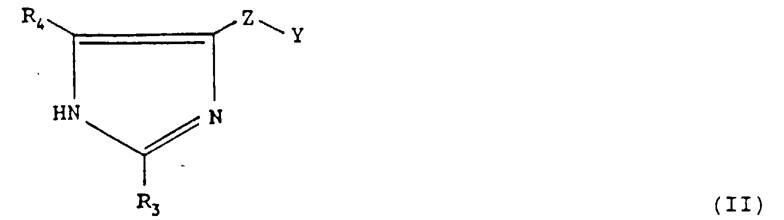 wherein Y represents Br, OH, or O-alkyl, with a thioureaderivative having the general formula: 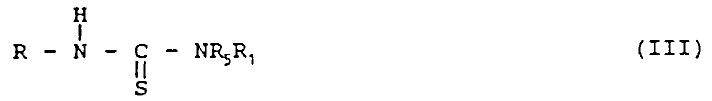 or condensation of a imidazole of formula II wherein Y represents NH₂, with a isothiourea-derivative having the general formula: 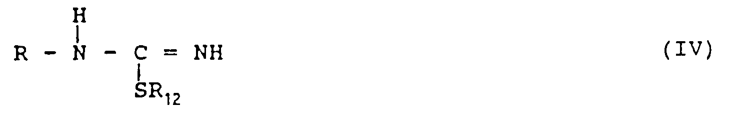 wherein in the formulas III and IV R represents hydrogen, (C₁-C₁₀)alkyl-, aryl(C₁-C₁₀)alkyl- or aryl, and R₁₂ represents (C₁-C₁₀)alkyl. As solvents polair solvents are used such as ethanol or propanol. The condensations are carried out at temperatures between roomtemperature and the boiling point of the solvents for between 30 minutes and 10 hours. Reactions take place in acid environment, e.g. hydrobromic acid, or in neutral environment. The obtained product can be processed in the usual way. If desired it is further possible to convert the obtained compounds of formula I in other compounds of formula I.

The following examples illustrate the synthesis of compounds of the present invention but are never intended to limit the scope thereof.

### EXAMPLE 1

Synthesis of N-benzyl-S-[2-(imidazol-4-yl)ethyl]isothiourea dipicrate (VUF 9028).

3.5 gram (13.7 mmol) 4(5)-(2-bromoethyl)imidazole.HBr and 2.3 gram N-benzylthiourea were refluxed for 60 hours in 30 ml ethanol. The ethanol was evaporated and the product was purified by means of columnchromatography, using methanol/ethylacetate as eluent.

Subsequently the solvent was evaporated and the residue dissolved in methanol whereto 10 gram picric acid in methanol was added. After addition of water an oil was formed, which after stirring with water became solid. The solid matter with melting point 166.9-169.8°C was subsequently filtrated. The NMR-results of this compound are given in table 1.

### EXAMPLE 2

Synthesis of S-[3-(4(5)-imidazolyl)alkyl]-N-(2-(substituted)-arylalkyl)isothiourea-derivatives.

Analogous to the preparation method of VUF 9028 from example 1 a number of compounds were synthesized with the formula: 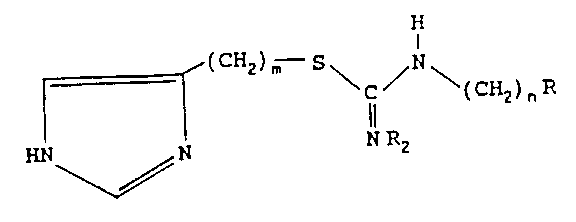 The meaning of n, m and R, the solvent of the condensation reaction and the melting points of the compounds are given in the table below. The NMR-results are given in table 1.

| Compound | R₂ | R | n | m | melt.point | salt | solvent |
|---|---|---|---|---|---|---|---|
| VUF 8397 | H | C₆H₅ | 0 | 2 | 174-176°C | 2HBr | 2-prop. |
| VUF 9029 | H | C₆H₅ | 2 | 2 | 177-185°C | 2HBr | eth. |
| VUF 9030 | H | C₆H₅ | 3 | 2 | 152-155°C | dipicr. | eth. |
| VUF 9031 | H | C₆H₅ | 4 | 2 | 136-139°C | 2HBr | eth. |
| VUF 9051 | CH₃ | C₆H₅ | 2 | 2 | 152-156°C | 2HBr | eth. |
| VUF 9107 | H | C₆H₅ | 1 | 3 | 155-160°C | 2HBr | eth. |
| VUF 9151 | H | C₆H₅ | 2 | 3 | 178-183°C | 2HBr | eth. |
| VUF 9152 | H | C₆H₅ | 3 | 3 | 177-184°C | 2HBr | eth. |
| VUF 9153 | H | 4-ClC₆H₄ | 1 | 3 | 200-205°C | 2HBr | eth. |
| VUF 9163 | H | c-C₆H₁₁ | 1 | 3 | 137-153°C | dipicr. | eth. |
| VUF 4571 | H | C₆H₅ | 4 | 3 | 112-134°C | dipicr. | eth. |
| VUF 4586 | H | 4-IC₆H₄* | 2 | 3 | 188-190°C | 2HBr | 2-prop. |

| | | | | | | | |
|---|---|---|---|---|---|---|---|
| * Radioactively labeled compound, e.g. for use as a tracer-molecule | | | | | | | |

### EXAMPLE 3

Synthesis of N-[2-(imidazol-4-yl)ethyl]-N'-phenyl guanidine dipicrate (VUF 9006).

### Step 1:

### Synthesis of S-ethyl-N-phenylisothiourea.

4 gram N-phenylisothiourea (33 mmol) and 5 ml ethylbromide were refluxed for 10 hours in ethanol. Again 5 ml ethylbromide was added. The reaction course was followed by thin layer chromatography (ethylacetate/petroleumether 3:7). Subsequently the solvent was evaporated and the residu crystallised from ethanol/ethylacetate.

### Step 2:

15 mmol histamine.2HCl was added to 30 mmol sodiumethanolate in ethanol (prepared by dissolving 30 mmol sodium in ethanol). Subsequently it was refluxed for one half hour, after which the mixture was cooled in ice and the formed NaCl was filtrated.

To the filtrate 15 mmol S-ethyl-N-phenylisothiourea was added. Next the reation mixture was refluxed for 35 hours (control with thin layer chromatography (ethylacetate/ petroleumether 1:1, saturated with ammonia)). Subsequently the solvent was evaporated and the residue dissolved in methanol. 35 mmol picric acid were added. The product was seperated by the addition of water and was subsequently crystallised from methanol/water. The melting point was 235-238°C.

Analogous to the synthesis of VUF 9006 N-[2-(imidazol-4-yl)ethyl]-N'-phenyl-ethylguanidine dipicrate (VUF 9007; meltingpoint 196-198°C) was prepared.
The NMR-resluts are given in table 1.

### EXAMPLE 4

### Synthesis of N-[2-(arylalkylthio)alkyl]-S-[3-(imidazol-4-yl)alkyl]isothiourea- and -guanidine-derivatives.

Analogous to example 1 compounds were synthesized having the formula: 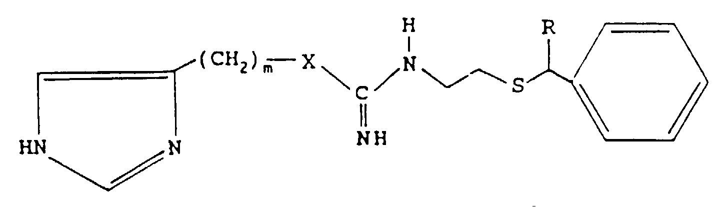 The meaning of the symbols m, X and R, the solvent of the condensation reaction and the melting points of the compounds are given in the table below. The NMR-results are given in table 1.

| Compounds | m | X | R | melting point | salt | solvent |
|---|---|---|---|---|---|---|
| VUF 8404 | 2 | S | H | 233-235°C | 2HBr | 2-prop. |
| VUF 8405 | 3 | NH | H | 145-148°C | dipicr. | ethanol |
| VUF 8409 | 2 | S | C₆H₅ | 106-109°C | dipicr. | ethanol |
| VUF 8414 | 3 | S | H | 126-133°C | dipicr. | ethanol |

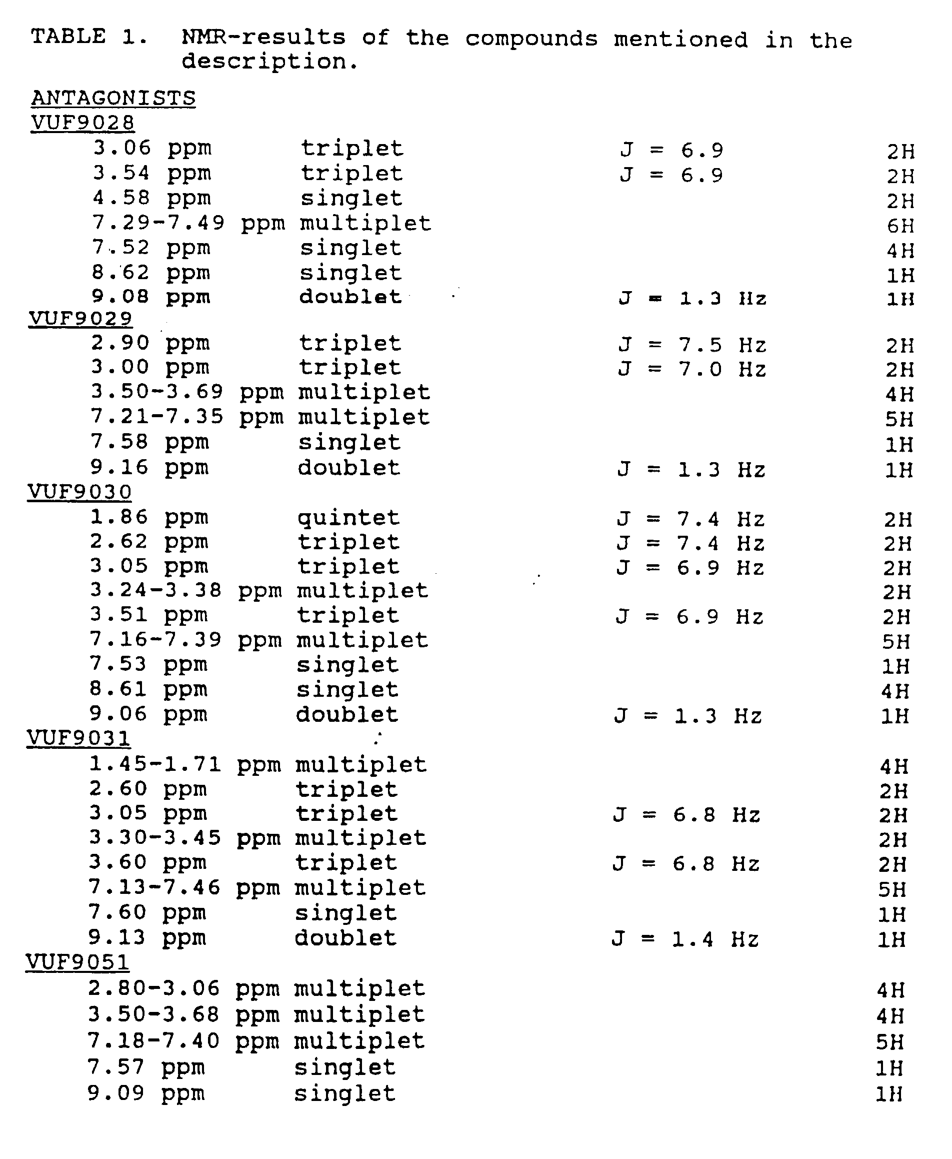

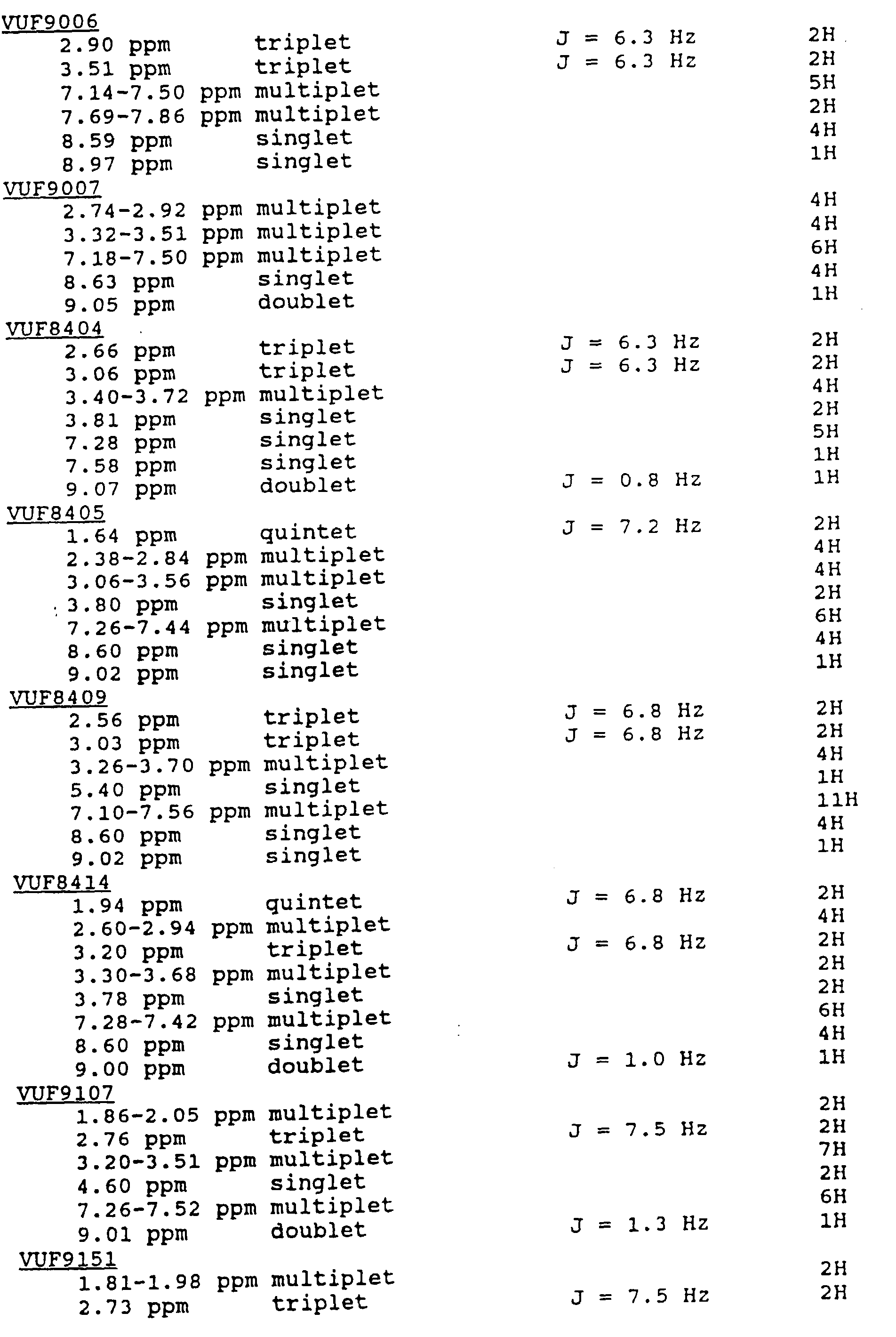

### Pharmacological experiments

The agonistics and antagonistics activities on the H₃-receptor of the various compounds were determined compared to histamine. The testmethods used therefor are described in Van der Werf et al., Agents and Actions 20, 3/4 (1987) p. 239-243 and Menkveld et al., European Journal of Pharmacology, 186 (1990) p. 343-347.

The results of the experiments are given in the tables below. pD₂ is the negative logarithm of the concentration of the testcompound at which 50% agonistic activity was measured. pA₂ is the negative logarithm of the concentration of the testcompound at which the concentration of the agonist had to be doubled to obtain the same effect as obtained when the antagonist was absent.

Pharmaceutical compositions, comprising compounds of formula I as defined in claim 19 as the active ingredient for therapeutically influencing the human and animal histaminergic system have the form of powders, suspensions, solutions, sprays, emulsions, unguents or creams and can be used for local application, intranasal, rectal, vaginal and also for oral or parenteral (intravenous, intradermal, intramuscular, intrathecal etc.) administration. Such compositions can be prepared by combining (i.e. by mixing, dissolving etc.) of the active compound of formula I in the form of a free acid or salt with farmaceutically acceptable excipients with neutral character (such as aquous or non-aquous solvents, stabilizers, emulsifiers, detergents, additives), and further if neccesary colouring agents and flavouring agents. The concentration of the active ingredient in a farmaceutical composition can vary between 0.1% and 100%, depending on the nature of the influence and the method of administration. The dose of the active ingredient that is administered can further be varied between 0.1 mg and 100 mg per kg bodyweight.

**TABLE 2.**

| Antagonistic activity | | |
|---|---|---|
| Compound | pA₂ | testmethod |
| VUF 8397 | 7.0 | ratcortex |
| VUF 9028 | 7.8 | ileum guinea pig |
| VUF 9029 | 8.0 | ileum guinea pig |
| VUF 9030 | 7.6 | ileum guinea pig |
| VUF 9031 | 7.7 | ileum guinea pig |
| VUF 9051 | 7.8 | ileum guinea pig |
| VUF 9006 | 5.8 | ileum guinea pig |
| VUF 9007 | 6.3 | ileum guinea pig |
| VUF 8404 | 7.4 | ileum guinea pig |
| VUF 8405 | 7.9 | ileum guinea pig |
| VUF 8409 | 6.6 | ileum guinea pig |
| VUF 8414 | 8.6 | ileum guinea pig |
| VUF 9107 | 8.8 | ileum guinea pig |
| VUF 9151 | 8.8 | ileum guinea pig |
| VUF 9152 | 8.3 | ileum guinea pig |
| VUF 9153 | 9.9 | ileum guinea pig |
| VUF 9163 | 8.8 | ileum guinea pig |
| VUF 4571 | 8.4 | ileum guinea pig |
| VUF 4586 | 9.2 | ileum guinea pig |

## Claims

1. Use of compounds of formula I 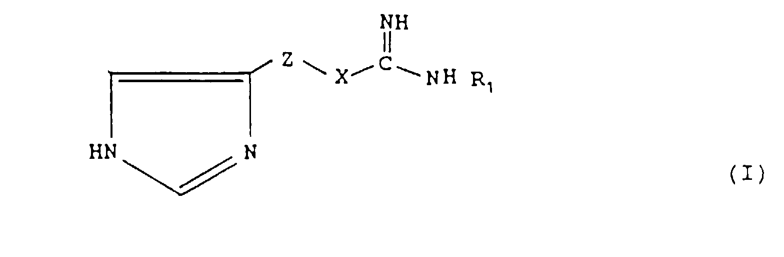 wherein
X is NH or S
Z is a group of the formula (CH₂)ₘ,
a) wherein m is 2 or 3 if R₁ is a group of the formula -(CH₂)ₙR₁₀, wherein R₁₀ is aryl and n ≧ 1 ; or
b) wherein m is 1, 2 or 3 if R₁ is a group of the formula 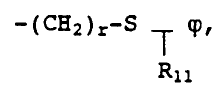 wherein ϕ is aryl, r is 1, 2 or 3 and R₁₁ is hydrogen, (C₁-C₁₀)alkyl or aryl;
wherein aryl is phenyl, naphtyl, pyridyl; or of the compounds :
S-[2-(imidazol-4-yl)ethyl]-N-(2-phenylethyl)isothiourea;
N-cyclohexylmethyl-S-[3-(4(5)-imidazolyl)propyl]isothiourea;
S-[3-(4(5)-imidazolyl)propyl]-N-(4-chloro-benzyl)isothiourea; and
S-[3-(4(5)-imidazolyl)propyl]-N-4-iodophenylethyl)isothiourea
for the preparation of a pharmaceutical composition having antagonistic activity on the histamine H₃-receptor.

2. Use as claimed in claim 1, wherein the derivative is N-benzyl-S-[3-(4(5)-imidazolyl)propyl]isothiourea.

3. Use as claimed in claim 1, wherein the derivative is S-[3-(4(5)-imidazolyl)propyl]-N-(2-phenylethyl) isothiourea.

4. Use as claimed in claim 1, wherein the derivative is S-[3-(4(5)-imidazolyl)propyl]-N-(3-phenylpropyl) isothiourea.

5. Use as claimed in claim 1, wherein the derivative is S-[3-(4(5)-imidazolyl)propyl]-N-(4-phenylbutyl) isothiourea.

6. Use as claimed in claim 1, wherein the derivative is N-[2-(benzylthio)ethyl]-S-[3-(imidazol-4-yl)propyl]isothiourea.

7. S-[2-(imidazol-4-yl) ethyl]-N-(2-phenylethyl)isothiourea.

8. N-benzyl-S-[3-(4(5)-imidazolyl)propyl]isothiourea.

9. S-[3-(4(5)-imidazolyl)propyl]-N-(2-phenylethyl)-isothiourea.

10. S-[3-(4(5)-imidazolyl)propyl]-N-(3-phenylpropyl)isothiourea.

11. S-[3-(4(5)-imidazolyl)propyl]-N-(4-phenylbutyl)isothiourea.

12. S-[3-(4(5)-imidazolyl)propyl]-N-(4-chlorobenzyl)isothiourea.

13. N-cyclohexylmethyl-S-[3-(4(5)-imidazolyl)propyl]isothiourea.

14. S-[3-(4(5)-imidazolyl)propyl]-N-(4-iodophenylethyl)isothiourea.

15. N-[2-(benzylthio)ethyl]-S-[3-(imidazol-4-yl)propyl]isothiourea.

16. Pharmaceutical composition having antagonistic activity on the histamine H₃-receptor comprising a suitable excipient and as the active ingredient a compound as claimed in claims 7-15 or a pharmacologically acceptable salt thereof.

17. Method for preparing imidazole-derivatives as claimed in any one of the claims 7-15, by condensation of an imidazole-derivative of the general formula: 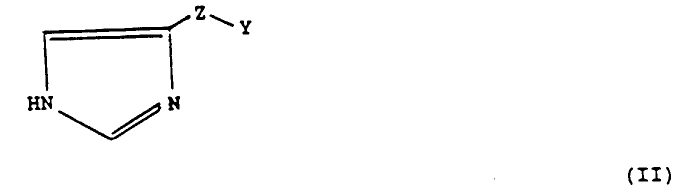 wherein Y represents Br, OH, or O-alkyl and Z is as defined in claim 1 ; and a thioureaderivative of the general formula: 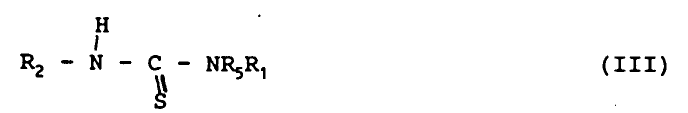 wherein R₁, R₂ and R₅ are as defined in claim 1.

18. Method for preparing imidazole-derivatives as claimed in any one of the claims 7-15, by condensation of an imidazole-derivative of the general formula: 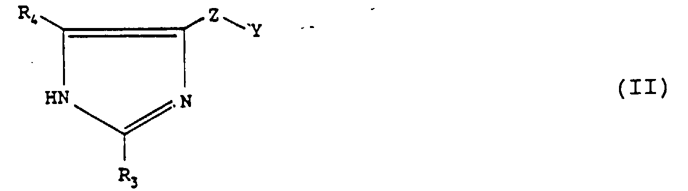 wherein Y represents NH₂; and an isothioureaderivative of the general formula 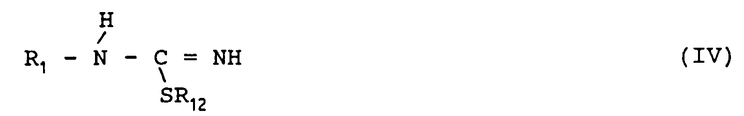 wherein R₁ has the same meaning as indicated in claim 1 R₁₂ represents (C₁-C₁₀)alkyl.

## Patentansprüche

1. Verwendung von Verbindungen der Formel I 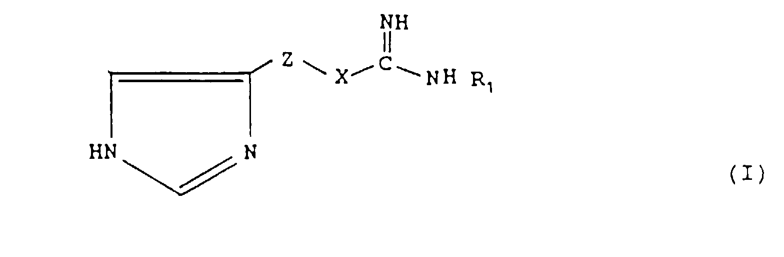 wobei
X für NH oder S steht,
Z für eine Gruppe der Formel (CH₂)ₘ steht,
a) wobei m gleich 2 oder 3 ist,
wenn R₁ für eine Gruppe der Formel -(CH₂)ₙR₁₀ steht, wobei R₁₀ für eine Arylgruppe steht und n ≥ 1 ist; oder
b) wobei m gleich 1, 2 oder 3 ist,
wenn R₁ für eine Gruppe der Formel 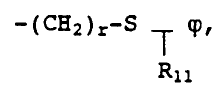 steht,
wobei ϕ für eine Arylgruppe steht,
r gleich 1, 2 oder 3 ist und R₁₁ für einen Wasserstoff, eine (C₁-C₁₀)-Alkyl- oder eine Arylgruppe steht;
wobei die Arylgruppe für eine Phenyl-, Naphthyl-, Pyridylgruppe steht; oder
der Verbindungen:
S-[2-(imidazol-4-yl)ethyl]-N-(2-phenylethyl)isothioharnstoff;
N-cyclohexylmethyl-S-[3-(4(5)-imidazolyl)propyl]isothioharnstoff;
S-[3-(4(5)-imidazolyl)propyl]-N-(4-chloro-benzyl)isothioharnstoff; und
S-[3-4(5)-imidazolyl)propyl]-N-4-iodophenylethyl)isothioharnstoff
zur Herstellung einer pharmazeutischen Zusammensetzung mit einer antagonistischen Wirkung auf den Histamin H₃-Rezeptor.

2. Verwendung, wie in Anspruch 1 beansprucht, wobei der Abkömmling N-benzyl-S-[3-(4(5)-imidazolyl)propyl]isothioharnstoff ist.

3. Verwendung, wie in Anspruch 1 beansprucht, wobei der Abkömmling S-[3-(4(5)-imidazolyl)propyl]-N-(2-phenylethyl)isothioharnstoff ist.

4. Verwendung, wie in Anspruch 1 beansprucht, wobei der Abkömmling S-[3-(4(5)-imidazolyl)propyl]-N-(3-phenylpropyl)isothioharnstoff ist.

5. Verwendung, wie in Anspruch 1 beansprucht, wobei der Abkömmling S-[3-(4(5)-imidazolyl)propyl]-N-(4-phenylbutyl)isothioharnstoff ist.

6. Verwendung, wie in Anspruch 1 beansprucht, wobei der Abkömmling N-[2-(benzylthio)ethyl]-S-[3-(imidazol-4-yl)propyl]isothioharnstoff ist.

7. S-[2-(imidazol-4-yl)ethyl]-N-(2-phenylethyl)-isothioharnstoff.

8. N-benzyl-S-[3-4(5)-imidazolyl)propyl]isothioharnstoff.

9. S-[3-(4(5)-imidazolyl)propyl]-N-(2-phenylethyl)-isothioharnstoff.

10. S-[3-(4(5)-imidazolyl)propyl]-N-(3-phenylpropyl)isothioharnstoff.

11. S-[3-(4(5)-imidazolyl)propyl]-N-(4-phenylbutyl)isothioharnstoff.

12. S-[3-(4(5)-imidazolyl)propyl]-N-(4-chlorobenzyl)isothioharnstoff.

13. N-cyclohexylmethyl-S-[3-(4(5)-imidazolyl)propyl]isothioharnstoff.

14. S-[3-(4(5)-imidazolyl)propyl]-N-(4-iodophenylethyl)isothioharnstoff.

15. N-[2-(benzylthio)ethyl]-S-[3-(imidazol-4-yl)propyl]isothioharnstoff.

16. Pharmazeutische Zusammensetzung mit einer antagonistischen Wirkung auf den Histamin H₃-Rezeptor, die einen geeigneten Arzneimittelträgerstoff und als aktive Komponente eine Verbindung, wie in den Ansprüchen 7-15 beansprucht, oder ein pharmakologisch akzeptables Salz dieser Verbindungen enthält.

17. Verfahren zur Herstellung von Imidazolabkömmlingen, wie in einem der Ansprüche 7-15 beansprucht, durch Kondensation eines Imidazolabkömmlings der allgemeinen Formel: 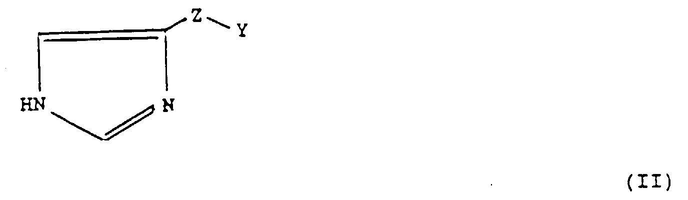 wobei Y für Br, OH oder eine O-Alkylgruppe steht und Z so, wie in Anspruch 1 definiert, ist ; und eines Thioharnstoffabkömmlings der allgemeinen Formel 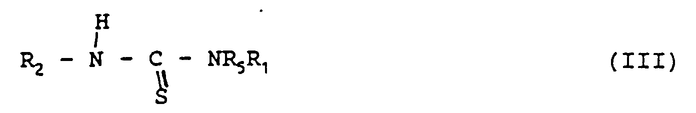 wobei R₁, R₂ und R₅ so, wie in Anspruch 1 definiert, sind.

18. Verfahren zur Herstellung von Imidazolabkömmlingen so wie in einem der Ansprüche 7-15 beansprucht, durch Kondensation eines Imidazolabkömmlings mit der allgemeinen Formel: 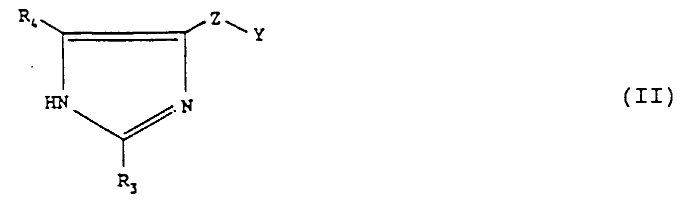 , wobei Y für NH₂ steht; und eines Isothioharnstoffabkömmlings mit der allgemeinen Formel 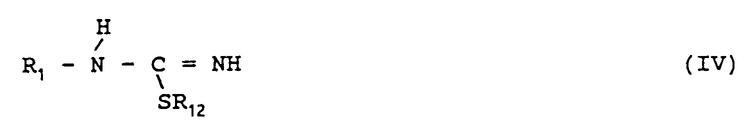 , wobei R₁ die gleiche Bedeutung wie in Anspruch 1 hat und R₁₂ für eine (C₁-C₁₀)-Alkylgruppe steht.

## Revendications

1. Utilisation de composés de formule I : 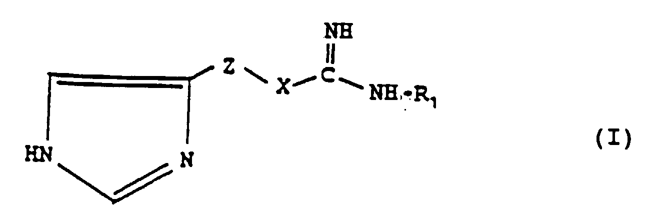 dans laquelle :
X est NH ou S
Z est un groupe de formule (CH₂)ₘ,
a) où m vaut 2 ou 3 si R₁ est un groupe de formule -(CH₂)ₙR₁₀, où R₁₀ est un groupe aryle et n≥1 ; ou
b) où m vaut 1, 2 ou 3 si R₁ est un groupe de formule 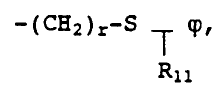
où ϕ est un radical aryle, r vaut 1, 2 ou 3 et R₁₁ est un hydrogène ou un groupe alkyle en C₁ à C₁₀ ou aryle;
où le radical aryle est un groupe phényle, naphtyle, pyridyle ; ou
des composés suivants :
S-[2-(imidazole-4-yl)éthyl]-N-(2-phényléthyl)iso-thiourée,N-cyclohexylméthyl-S-[3-(4(5)-imidazolyl)-propyl]isothiourée, S-[3-(4(5)-imidazolyl)propyl]-N-(4-chlorobenzyl)isothiourée, et S-[3-(4(5)-imidazolyl)-propyl]-N-4-iodophényléthyl)isothiourée, pour préparer une composition pharmaceutique ayant une activité antagoniste sur le récepteur de l'histamine H₃.

2. Utilisation selon la revendication 1, dans laquelle le dérivé est la N-benzyl-S-[3-(4(5)-imidazolyl)propyl]isothiourée.

3. Utilisation selon la revendication 1, dans laquelle le dérivé est la S-[3-(4(5)-imidazolyl)propyl]-N-(2-phényléthyl)isothiourée.

4. Utilisation selon la revendication 1, dans laquelle le dérivé est la S-[3-(4(5)-imidazolyl)propyl]-N-(3-phénylpropyl)isothiourée.

5. Utilisation selon la revendication 1, dans laquelle le dérivé est la S-[3-(4(5)-imidazolyl)propyl]-N-(4-phénylbutyl)isothiourée.

6. Utilisation selon la revendication 1, dans laquelle le dérivé est la N-[2-(benzylthio)éthyl]-S-[3-(imidazole-4-yl)propyl]isothiourée.

7. S-[2-(imidazole-4-yl)éthyl]-N-(2-phényléthyl)isothiourée.

8. N-benzyl-S-[3-(4(5)-imidazolyl)propyl]isothiourée.

9. S-[3-(4(5)-imidazolyl)propyl]-N-(2-phényléthyl)isothiourée.

10. S-[3-(4(5)-imidazolyl)propyl]-N-(3-phénylpropyl)isothiourée.

11. S-[3-(4(5)-imidazolyl)propyl]-N-(4-phénylbutyl)isothiourée.

12. S-[3-(4(5)-imidazolyl)propyl]-N-(4-chlorobenzyl)isothiourée.

13. N-cyclohexylméthyl-S-[3-(4(5)-imidazolyl)propyl]isothiourée

14. S-[3-(4(5)-imidazolyl)propyl]-N-(4-iodophényléthyl)isothiourée.

15. N-[2-benzylthio)éthyl]-S-[3-(imidazole-4-( yl)propyl]isothiourée.

16. Composition pharmaceutique ayant une activité antagoniste sur le récepteur de l'histamine H₃, qui comprend un excipient approprié, et, en tant que principe actif, un composé selon les revendications 7 à 15, ou un sel acceptable d'un point de vue pharmacologique de ce composé.

17. Procédé pour préparer des dérivés de l'imidazole selon l'une quelconque des revendications 7 à 15, par condensation d'un dérivé de l'imidazole de formule générale : 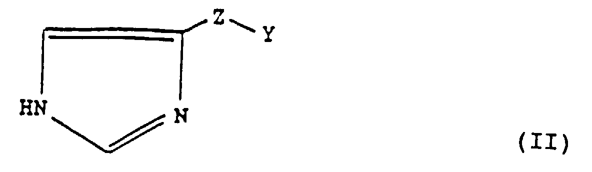 dans laquelle Y représente Br, OH, ou un groupe o-alkyle, et Z est tel que défini dans la revendication 1;
et d'un dérivé de thiourée de formule générale : 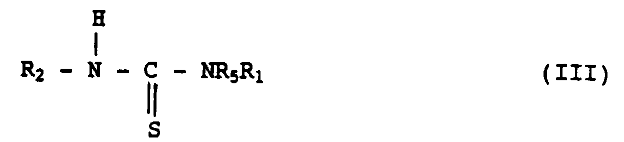 dans laquelle R₁, R₂ et R₅ sont tels que définis dans la revendication 1.

18. Procédé pour préparer des dérivés de l'imidazole selon l'une quelconque des revendications 7 à 15, par condensation d'un dérivé de l'imidazole de formule générale : 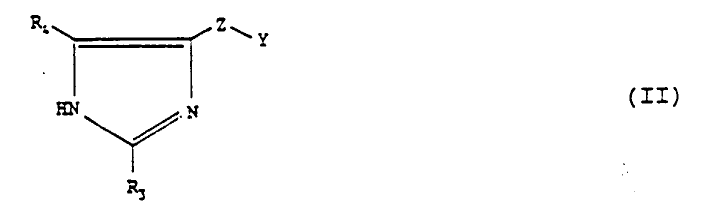 dans laquelle Y représente NH₂ ; et d'un dérivé de l'isothiourée de formule générale : 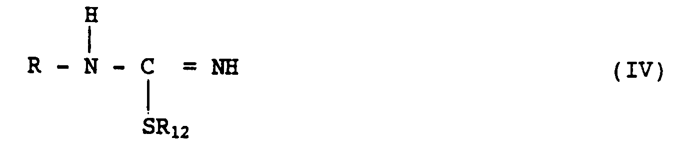 dans laquelle R₁ a les mêmes significations que dans la revendication 1, et R₁₂ représente un groupe alkyle en C₁ à C₁₀.
